# EUROPEAN PATENT APPLICATION

(11) **EP 4 607 455 A1**
(43) Date of publication of application: **27.08.2025**
(21) Application number: 24158945.6
(22) Date of filing: 21.02.2024
(51) Int. Cl.: G06T 7/00, A61B 5/00

(54) **PROCESSING HYPERSPECTRAL IMAGES OF SKELETAL JOINTS**

(71) Applicant: Afara, Isaac, 70420 Kuopio (FI); Mirhashemi, Arash, 70200 Kuopio (FI); Nippolainen, Ervin, 70840 Kuopio (FI)
(72) Inventor: Afara, Isaac, 70420 Kuopio (FI); Mirhashemi, Arash, 70200 Kuopio (FI); Nippolainen, Ervin, 70840 Kuopio (FI)
(74) Representative: Berggren Oy

(57) **Abstract**

Methods, an apparatus, and a computer program product for processing hyperspectral images of joints are disclosed. A method comprises: obtaining (200) a hyperspectral image depicting at least a part of a skeletal joint of a subject, and processing (202) the hyperspectral images by a trained machine learning model, wherein the processing comprises: predicting (204) biomarker values for pixels of the hyperspectral image, and generating (206) a biomarker image comprising the predicted biomarker values; wherein the machine learning model has been trained using a training data set to process hyperspectral images of skeletal joints, wherein the training data set comprises a plurality of training images depicting skeletal joints of training subjects, wherein, for each skeletal joint of the training data set, the training data set comprises: at least one hyperspectral image of at least a part of the joint, and at least one corresponding ground truth image based on a non-hyperspectral image of at least the part of the joint, wherein the ground truth image comprises one or more ground truth biomarker values for at least the part of the joint also included in at least one hyperspectral image.

## Description

### Technical Field

The present solution generally relates to computer-implemented methods, an apparatus, and a computer program product for processing hyperspectral images of skeletal joints.

### Background

A growing number of patients struggle with osteoarthritis (OA) - a degenerative joint disease associated with pain and immobility. Although OA is most prevalent among seniors, it can also develop in young people with sports and trauma-related injuries to the joint. In these cases, any post-treatment joint instability can result in trauma-induced OA in the long run. The mechanism of OA progression is poorly understood; thus, no ideal technique exists for monitoring and treatment. The socio-economic impacts of OA and its direct and indirect costs have made OA a major public health issue worldwide, particularly in Finland, with one of the highest knee and hip replacement surgeries in Europe.

Trauma-induced OA often initiates from injuries to the articular cartilage (AC) matrix. Thus, understanding its composition and structural integrity (i.e., tissue characterization) are useful for early-stage assessment of OA. The gold standard laboratory methods for characterizing AC are histological assessment and biomechanical testing, both of which are destructive to the tissue. Non-invasive imaging methods, such as X-ray and magnetic resonance (MR) imaging, provide limited diagnostic value due to their low tissue contrast and resolution. Polarized Light Imaging (PLI) and Phase-Contrast Imaging (PCI) techniques provide high spatial resolution. Contrast ratio PLI uses a polarizer-analyzer setup to register the changes in light polarization by the sample. PCI records the changes in the light's phase due to the difference in refractive index of distinct sample areas. However, these techniques are limited to assessment of histology sections. Reliable, sensitive, and non-destructive tissue characterization and monitoring methods are currently unavailable. Clinically, AC tissue integrity is assessed visually via arthroscopy. However, this method is subjective and poorly reproducible.

### Summary of the Invention

The scope of protection sought for various embodiments of the invention is set out by the independent claims. Various embodiments are disclosed in the dependent claims. The embodiments and features, if any, described in this specification that do not fall under the scope of the independent claims are to be interpreted as examples useful for understanding various embodiments of the invention.

According to a first aspect, a computer-implemented method for processing hyperspectral images of skeletal joints comprises: obtaining a hyperspectral image depicting at least a part of a skeletal joint of a subject, and processing the hyperspectral images by a trained machine learning model, wherein the processing comprises: predicting biomarker values for pixels of the hyperspectral image, and generating a biomarker image comprising the predicted biomarker values; wherein the machine learning model has been trained using a training data set to process hyperspectral images of skeletal joints, wherein the training data set comprises a plurality of training images depicting skeletal joints of training subjects, wherein, for each skeletal joint of the training data set, the training data set comprises: at least one hyperspectral image of at least a part of the joint, and at least one corresponding ground truth image based on a non-hyperspectral image of at least the part of the joint, wherein the ground truth image comprises one or more ground truth biomarker values for at least the part of the joint also included in at least one hyperspectral image.

According to a second aspect, a computer-implemented method for processing hyperspectral images of skeletal joints comprises: obtaining a training data set comprising a plurality of training images depicting skeletal joints of training subjects, wherein, for each skeletal joint of the training data set, the training data set comprises: at least one hyperspectral image of at least a part of the joint, and at least one corresponding ground truth image based on a non-hyperspectral image of at least the part of the joint, wherein the ground truth image comprises one or more ground truth biomarker values for at least the part of the joint also included in at least one hyperspectral image; training a machine learning model using the training data set to process hyperspectral images of skeletal joints, wherein the machine learning model is configured to process the hyperspectral images of skeletal joints by: predicting biomarker values for pixels of a hyperspectral image, and generating a biomarker image comprising the predicted biomarker values.

The predicting may comprise predicting each biomarker value in the biomarker image based on a plurality of pixels of the hyperspectral image.

The biomarker may comprise at least one of the following:
- thickness of articular cartilage,
- collagen orientation of articular cartilage,
- tissue composition such as proteoglycan content of articular cartilage and/or collagen content of articular cartilage,
- subchondral plate thickness,
- porosity of subchondral bone,
- mineral density of subchondral bone,
- subchondral bone volume fraction,
- water content,
- cartilage integrity scored with the Mankin system and/or the Osteoarthritis Research Society International system,
- one or more biomechanical properties such as instantaneous modulus, equilibrium modulus, and/or dynamic modulus.

The ground truth images of the training data may be based on non-hyperspectral images comprising one or more of the following:
- computed tomography images such as micro-computed tomography images,
- densitometry images,
- polarized light microscopy images,
- water content maps,
- biomechanical maps,
- cartilage integrity maps scored with the Mankin and/or Osteoarthritis Research Society International systems.

The ground truth images of the training data may be based on computed tomography images such as micro-computed tomography images, and the biomarker may comprise one or more of the following:
- thickness of articular cartilage,
- subchondral plate thickness,
- porosity of subchondral bone,
- mineral density of subchondral bone,
- subchondral bone volume fraction.

The ground truth images of the training data may be based on densitometry images, and the biomarker may comprise proteoglycan content of articular cartilage.

The ground truth images of the training data may be based on polarized light microscopy images, and the biomarker may comprise collagen orientation of articular cartilage.

Pixels of the ground truth images of the training data may comprise ground truth biomarker values.

The machine learning model may comprise a neural network, preferably a convolutional neural network.

The machine learning model may comprise a modified U-Net wherein the final layers of the modified U-Net include a final convolutional layer followed by a regression layer.

For each corresponding ground truth image and hyperspectral image of the training data, the hyperspectral image and the ground truth image may be registered to the same coordinate system.

According to a third aspect, an apparatus is configured to perform the method of any one of claims 1 to 12.

The apparatus may comprise one or more processors, one or more memories including computer program code, the one or more memories and the computer program code configured to, with the one or more processors, cause the apparatus to perform the method of any one of claims 1 to 12.

According to a fourth aspect, a computer program product comprises computer program code configured to, when executed by at least one processor, cause an apparatus or a system to perform the method of any one of claims 1 to 12.

### Brief Description of the Drawings

FIG. 1 illustrates an embodiment of an apparatus for processing hyperspectral images;
FIG. 2 is a flow chart illustrating a first method for processing hyperspectral images;
FIG. 3 is a flow chart illustrating a second method for processing hyperspectral images;
FIG. 4 is a flow chart illustrating embodiments of the first and second methods for processing hyperspectral images;
FIG. 5 shows a bovine knee joint used for obtaining example training data;
FIG. 6 shows a micro-CT image slice with articular cartilage tissue attached to bone;
FIG. 7 depicts an example architecture of a U-net convolutional neural network for image segmentation;
FIG. 8 depicts an example architecture of a U-net convolutional neural network for image regression.

### Detailed Description of the Invention

The following description and drawings are illustrative and are not to be construed as unnecessarily limiting. The specific details are provided for a thorough understanding of the disclosure. However, in certain instances, well-known or conventional details are not described in order to avoid obscuring the description.

The inventors have found spectroscopy to be sensitive to early-stage changes in AC tissue properties before visible signs of degeneration appear. Hyperspectral imaging (HSI), which can be considered the imaging counterpart of spectroscopy, provides an image data cube with two spatial dimensions and a spectral dimension that presents the reflectance spectrum of each pixel location, i.e., each pixel of a hyperspectral image provides the spectrum at a certain point in space. HSI provides reflectance spectra that entail depth-wise optical properties of the sample, which is a function of the wavelength-dependent penetration depth of light into the tissue. Thus, HSI can provide a signal that correlates with biomarkers of the AC tissue in images taken from a sample's surface. The hyperspectral images also contain spatial information about the spread of the damage on the tissue surface. This spatial information provides better insight into the spread of the damage and properties of the tissues in comparison to point measurements. The spatial nature of hyperspectral images also provides anatomical specificity and improves the spatial correlation between sequential measurements, in contrast with point measurements.

Thus, HSI is a suitable candidate for non-destructive computational characterization of AC tissue e.g., via statistical estimation. Applicable computational models can be based on machine learning, such as neural network architectures. The methods, apparatus, and computer program product disclosed herein are integrable with an arthroscopic pipeline in a real-time imaging setup for live mapping of damaged AC tissue during a surgical procedure. This configuration can delineate the anatomical location of the damage and its severity. Consequently, this information may be used in evaluating and monitoring tissues with osteoarthritis, as well as to provide personalized and optimal treatment plan for patients.

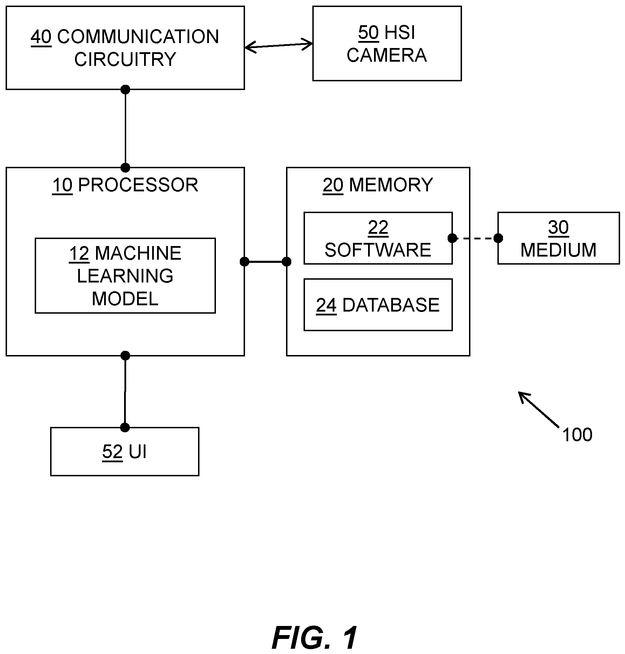

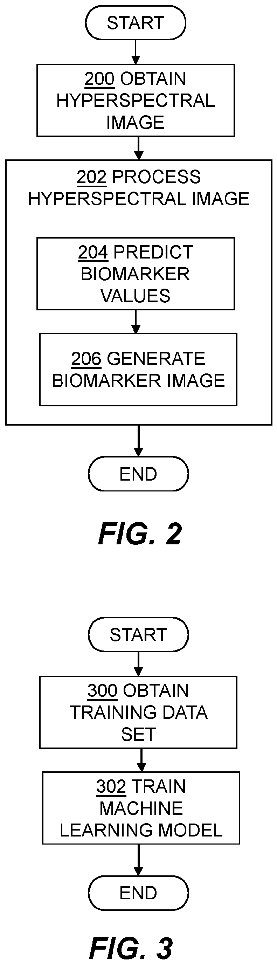

FIG. 1 is a schematic diagram depicting an embodiment of an apparatus 100 for processing hyperspectral images. The apparatus 100 may be any computing device, such as a general-purpose computer (e.g. a server computer), or an application-specific computing device such as an arthroscopic probe. The apparatus is configured to perform a first method of processing hyperspectral images illustrated in FIG. 2, and/or a second method of processing hyperspectral images illustrated in FIG. 3, and/or any of their embodiments. Especially when configured to perform the method of FIG. 2, the apparatus 100 may form part of a medical imaging or surgical system, particularly one suitable for use in assessing joint tissue integrity e.g. in arthroscopic surgery. In this case, the apparatus may be or form part of an arthroscope.

The apparatus may be implemented in the form of a virtual machine. The methods of FIG. 2 and/or FIG. 3, described in more detail below, may be executed by the apparatus 100 as a containerized application using operating system (OS) -level virtualization. Alternatively, or additionally, the method may be executed in a distributed computing environment and/or as a cloud service. In these situations, the apparatus 100 may be a computing unit of the distributed computing environment or the cloud service.

The apparatus 100 includes a processor 10. The processor may include a central processing unit (CPU) and/or a graphics processing unit (GPU) for processing image and/or video data. The apparatus 100 further includes a memory 20. The memory 20 may comprise various types of memories, such as a non-volatile memory and a random access memory (RAM). The memory contains computer program code 22 for controlling the apparatus. The skilled person understands that a different number and/or different types of processors and/or memories may be included in the apparatus 100.

The apparatus 100 comprises a communication circuitry 40 for communicating with other devices. The communication may be implemented via wired and/or wireless networks, such as an IOT network, a cellular network, or a personal area network (PAN), for example. When the apparatus is a server computer and/or part of the distributed computing environment or the cloud service, the apparatus 100 may be located in a data center and accessible via the network through the communication circuitry 40. The skilled person understands that the communication circuitry 40 may be omitted when the apparatus 100 otherwise has access to the information and/or resources needed for performing the method of FIG. 2 and/or FIG. 3.

The apparatus 100 further comprises, or is coupled to, a HSI camera 50. In the embodiment of FIG. 1 the apparatus 100 is coupled to the HSI camera 50 via the communication circuitry 40, but the HSI camera can alternatively be included as a part of the apparatus 100. The HSI camera comprises e.g. charge-coupled device (CCD) sensors, ordinary optical components (non-spectral and non-moving) known to the skilled person, and narrow-band light sources. The memory 20 of the apparatus 100 may contain a database 24 for storing hyperspectral images captured by the HSI camera 50 or by another HSI camera.

A standard HSI camera is suitable for the apparatus when it is configured to perform the method of FIG. 2 and/or FIG. 3. For both methods, the same HSI camera system, light source configuration, optics (distance to subject, lens parameters, etc.), wavelength range, resolution, and post-processing steps may be used. However, it is not necessary to maintain the same setup in respect of some or any of these parameters. The skilled person is aware of post-processing strategies to minimize the effect of the differences that different setups may cause. For example, the effect of using a different light source is automatically minimized when the collected HSI data are normalized by the light source spectrum.

The skilled person understands that the HSI camera 50 may be omitted when the apparatus 100 otherwise has access to the hyperspectral images needed for performing the method of FIG. 2 and/or FIG. 3. For example, the apparatus may obtain the hyperspectral images form the database 24 of the memory 20.

The apparatus 100 further comprises, or is coupled to, a user interface (UI) 52. In the embodiment of FIG. 1 the UI 52 is a part of the apparatus 100, but the UI can alternatively be coupled to the apparatus 100 e.g. via the communication circuitry 40. The user interface 52 may comprise various input and/or output devices, such as a display, a touch pad, a touch screen, a speaker, a microphone, and/or a haptic output device. Again, the skilled person understands that the UI 52 may be omitted e.g. when the apparatus 100 receives its inputs and provides its outputs via the communication circuitry 40, for example.

As mentioned above, the apparatus is configured to perform a first method of processing hyperspectral images illustrated in FIG. 2, and/or a second method of processing hyperspectral images illustrated in FIG. 3, and/or any of their embodiments. When executed by the processor 10 of the apparatus, the computer program code 22 included in the memory 20 of the apparatus causes the apparatus 100 to perform the first and/or second method, and/or any of their embodiments. The computer program code 22 may be stored on a (optionally non-transitory) computer-readable medium 30 in the form of a computer program product.

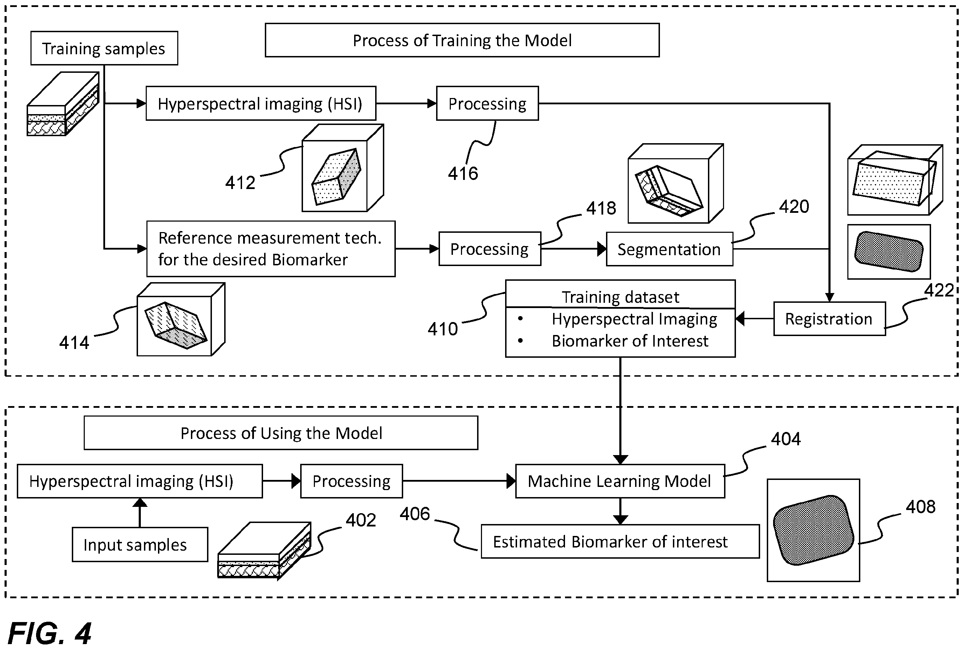

With reference to FIG. 2 and FIG. 4, the first method comprises obtaining 200 a hyperspectral image depicting at least a part of a skeletal joint 402 of a subject. The image may be obtained as a single image, or from a sequence of images or a video captured by a HSI camera. With reference to FIG. 1, the apparatus 100 may obtain the hyperspectral image from the database 24 of the memory 20 of the apparatus 100, from the HSI camera 50, or from another device via the communication circuitry 40, for example. For use during arthroscopic surgery, the HSI camera 50 is preferably integrated within an arthroscope, and the image(s) captured by the HSI camera is obtained.

Referring again to FIG. 2 and FIG. 4, the first method further comprises processing 202 the hyperspectral images by a trained machine learning model 404. The processing 202 comprises predicting 204 biomarker values 406 for pixels of the hyperspectral image, and generating 206 a biomarker image 408 comprising the predicted biomarker values 406. The machine learning model, its configuration and training, and example biomarkers will be described in more detail when discussing the second method of FIG. 3.

The first method may further comprise outputting the generated biomarker image. With reference to FIG. 1, the outputting may comprise writing the biomarker image to a memory, such as the memory 20 of the apparatus 100. Alternatively, or additionally, the outputting may comprise sending the biomarker image e.g. via the communication circuitry 40. Alternatively, or additionally, the outputting comprises outputting the biomarker image by the UI 52 of the apparatus 100 e.g. via a display of the UI. Aspects of visualizing the biomarker image are described in more detail later.

HSI is agile, especially in systems that are based on multispectral imaging, spectral filtering, or band selection, which can perform snapshot images. The first method can provide real-time or live mapping (e.g. video) of estimated biomarkers, and improve the temporal correlation between sequential measurements.

The machine learning model and its training will now be described in more detail with reference to FIG. 3 and FIG. 4. The second method of processing hyperspectral images comprises obtaining 300 a training data set 410. The training data set 410 comprises a plurality of training images depicting skeletal joints of training subjects. Each of the skeletal joints is a skeletal joint of a certain training subject. Multiple skeletal joints of the training subject may also be included in the training data set. For each skeletal joint included in the training data set, the training data set comprises at least one hyperspectral image 412 of at least a part of the joint, and at least one corresponding ground truth image based on a non-hyperspectral image 414 of at least the same part of the joint. The ground truth image includes one or more ground truth biomarker values for use in the training of the machine learning model. The ground truth biomarker values are included for at least the part of the joint also included in at least one hyperspectral image, i.e., the part of the joint that is depicted in both the hyperspectral image and the non-hyperspectral image. A plurality of hyperspectral and/or ground truth images based on non-hyperspectral images for each joint may be included in the training data. In this case, relevant steps of preparing the training data and training the machine learning model can be repeated accordingly.

The second method further comprises training 302 the machine learning model 404 using the training data set 410. The machine learning model is configured to process hyperspectral images of skeletal joints as described above in relation to the first method, and the training optimizes the model for this purpose. With reference to FIG. 1, the memory 20 may be configured to store the training data and the apparatus 100 may obtain the training data from e.g. the database 24 of the memory 20 of the apparatus 100. Alternatively, or additionally, the apparatus 100 may obtain the training data from another device via the communication circuitry 40, for example. As mentioned above, when the apparatus 100 is configured to perform the method of FIG. 3, it is evident that at least the HSI camera 50 and the UI 52 may be omitted from the apparatus 100.

For the non-hyperspectral images on which the ground truth images are based, any images indicative of a desired biomarker related to skeletal joints are generally suitable. The machine learning model will, through its training, learn to draw connections between the biomarkers included in the ground truth images with the features of the hyperspectral images. The table below shows examples of non-hyperspectral image types or imaging modalities and on the same row, respective biomarkers that may be obtained using that image type or modality. It is appreciated by the skilled person that the biomarkers given in the table may also be obtained from some other non-hyperspectral images than the one on the respective row. It is also appreciated by the skilled person that the non-hyperspectral images given in the table may be used to obtain other biomarkers than those given on the respective row.

| **Non-hyperspectral image** | **Biomarker** |
|---|---|
| Computed tomography (CT) such as micro-computed tomography (micro-CT) | Thickness of articular cartilage, subchondral plate thickness, porosity of subchondral bone, mineral density of subchondral bone, subchondral bone volume fraction |
| (Digital) densitometry (DD) | Proteoglycan content of articular cartilage |
| Polarized light microscopy (PLM) | Collagen orientation of articular cartilage |
| Water content map | Water content |
| Biomechanical map | Biomechanical properties such as instantaneous modulus, equilibrium modulus, and/or dynamic modulus |
| Cartilage integrity map (Mankin, Osteoarthritis Research Society International (OARSI) | Cartilage integrity scored according to the Mankin system and/or the OARSI system |
| Biochemical map | Uronic acid, Proteoglycan content of articular cartilage |
| | Hydroxyproline, Collagen content of articular cartilage |

Any CT images may be suitable for the invention, but micro-CT images are particularly preferred. In general, the better the resolution of the image is the more suitable it is for the invention.

Uronic acid may be used as the biomarker itself, or it can be used as an intermediate based on which proteoglycan content of the articular cartilage is determined.

Hydroxyproline may be used as the biomarker itself, or it can be used as an intermediate based on which the collagen content of the articular cartilage is determined.

HSI has 2 spatial dimensions (that represents the sample surface XY plane) and 1 spectral dimension. The non-HSI image input to the model preferably also has the same 2 spatial dimensions (image surface). A 3rd dimension of the non-HSI image can be a scalar value, or a vector of values, possibly corresponding to different physical variables. The model can be trained to estimate the components of this vector all at once (multivariate model) or separate models can be used to estimate each component separately.

Preferably, the subject whose skeletal joints are studied using the trained machine learning model is a human subject. However, the training data may be, but need not be, acquired from subjects of the same species as the intended user group of the machine learning model. Training data obtained from one vertebrate species maybe suitable for training a machine learning model used for another vertebrate species, for example. The machine learning model may be initially trained with training data of one or more species different from the intended user species, and then fine-tuned and/or tested with training data of the intended user species. Further, the joints included in the training data may be, but need not be the same or of the same type as the joints for which the trained machine learning model is used. At least the following options and all their workable combinations are contemplated:
- Species (e.g., human, bovine) of training subjects and the subjects for whom the trained machine learning model is used is the same or different.
- The structural type(s) (e.g., articulating joint, fibrous joint, cartilaginous joint, synovial joint, facet joint) of the joints in the training data and the joint(s) processed by the trained machine learning model is the same or different.
- The anatomical type(s) (e.g., knee joint, hip joint, shoulder joint ...) of the joints in the training data and the joint(s) processed by the trained machine learning model is the same or different. When the anatomical type of the joints is the same, the structural type of the joints is consequently the same.

Differences in e.g. the species, the structural type, or the anatomical type between the training data and the intended subjects can be accounted for using transfer learning techniques. However, preferably, when the intended subjects are human subjects, the training is done using human samples. When the intended subjects are (racing) horses, training data from horses and/or ponies may be used.

The training data set contains pairs of a hyperspectral image and a corresponding ground truth image (biomarker map), each pair measured from the same subject and the same location of the same joint on the subject, and further registered together as described in more detail below. The training data can be from extracted tissue areas or in vivo measurements, accessed during surgical operation. The best practice is to have a large training set that is statistically representative of the tissue under investigation and contains examples from various scenarios that might be present in future inputs. For example, the data set preferably includes a range of samples from healthy, to mild, and severe damage levels.

Three example sets of training data for knee joints are provided below.

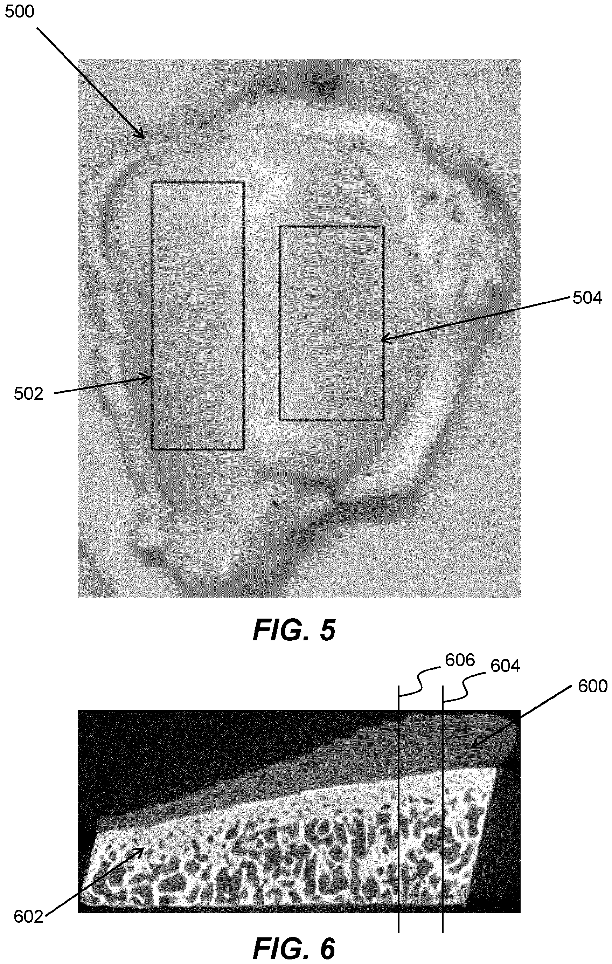

Referring to FIG. 5, the first training data set is from bovine knee joints 500 readily accessible from local abattoirs. Two rectangular AC-on-bone samples 502, 504 are extracted from each side of the patellar ridge. The patella provides a large flat area favorable for imaging. The first training data set may be used for training a machine learning model intended for bovines, more specifically for bovine knee joints.

The second training data set includes equine knee joints obtained from a veterinary hospital. They provide a non-flat geometry of more complex anatomical locations, such as femoral and tibial AC. The second training data set may be used for training a machine learning model intended for equines (e.g. racing horses), specifically equine knee joints.

The third training data set comprises human cadaver knee joints or samples obtained from patients undergoing joint replacement surgery. The third training data set may be used for training a machine learning model intended for humans, more specifically human knee joints.

An example data collection protocol carried out by the inventors for the bovine specimens is as follows. Bovine knee joint specimens are obtained from a local abattoir within 24 hours of slaughter and the patellae are surgically extracted. Referring to FIG. 5, from each patella 500, two rectangular samples 502, 504, are then extracted from the medial and the lateral compartments using a band saw. The samples are promptly prepped for micro-CT imaging, followed by biomechanical testing within the next 24 hours. Then, the samples are frozen and stored at -20 °C while accumulating the desired number of samples (e.g. 20 samples from 10 bovine joints). When all the samples are collected, they are allowed to thaw to room temperature and then transferred to a HSI facility to capture their hyperspectral images. The spectral range of the HSI covers the ultraviolet-A to near-infrared wavelengths (330nm to 1000nm).

When one or more analyses performed on the samples are destructive to the samples, the example protocol may include dividing the samples into two or more sets. When e.g. histology sectioning, biomechanical analysis, and/or water content analysis are to be performed on the samples, the following steps may be performed as part of the example protocol. After HSI, some (e.g. half) of the samples may be kept in ethylenediaminetetraacetic acid (EDTA) and formalin solution for decalcification to undergo histology sectioning, and the rest may be cut into a grid of small pieces of about 0.5 cm² to be weighed and dried for water content analysis. Dividing the samples into two sets only serves the purpose of obtaining both histology-based training data and water content training data in the context of the example protocol, as the histology analysis and the water content analysis are destructive to the samples.

The equine and human cadaver specimens may be subject to similar protocols.

The hyperspectral images of the training data are preferably imaged using a light source that is broadband to have a continuous spectral power distribution in the range of wavelengths of the HSI.

Example ways of determining the biomarkers provided in the above table are outlined below.

The thickness of the articular cartilage and the subchondral bone plate may be calculated by counting pixels with segment labels corresponding respectively to articular cartilage or subchondral bone in a segmented micro-CT image in the perpendicular direction to the tissue surface. A detailed example is provided further below.

Volume fraction of subchondral bone may be computed as the ratio of the subchondral bone volume, i.e., the number of voxels segmented as subchondral bone, to the volume of the total region being considered. The total region may be e.g. the whole sample under analysis.

Porosity may be calculated as the ratio of the volume of pores in the subchondral bone to the total volume of the subchondral bone in a (high-resolution) micro-CT image. Standard image processing techniques known to the skilled person may be used to distinguish the pores from the subchondral bone. A three or two-dimensional map can be achieved when the porosity is calculated locally and interpolated at each voxel or averaged in the direction perpendicular to the subchondral bone surface.

Mineral density of the subchondral bone can be calculated by calibrating the micro-CT image using the gray-scale values obtained for phantoms with known densities. Using a calibration curve, one can transform the gray-scale micro-CT image to the map of mineral density. Such calibration curves are available to the skilled person.

The biomechanical tests may be carried out with a mechanical testing system. The system may measure displacement (strain) and forces. It may have an indenter (for example a 0.5mm rod with spherical tip) which applies load on the cartilage. Different biomechanics modulus (e.g. instantaneous modulus, equilibrium modulus, and dynamic modulus) can be measured with the device based on the loading protocol of the testing system.

The following sets out an example protocol for processing the histology sections, which has been performed by the inventors on bovine cartilage-on-bone sample blocks. The sample blocks are cut to 4 to 6 smaller pieces depending on their length. Using a microtome, each piece is sectioned until its middle region. From the middle region several slices are cut. Half of slices are used for PLM and the rest are stained with Safranin-O for DD. From each set, the best slice is chosen which has no folding or cut in the tissue, and is imaged under microscope according to DD and PLM protocols known to the skilled person. Then, the resulting DD and/or PLM images are processed (including image stitching if needed) and analyzed to find the biomarkers. The biomarkers are considered locally, thus, no averaging for the whole section (a common practice in normal case) is performed. Finally, from the local points, and according to the location where the sections were taken in the sample block, the biomarker map is interpolated for the whole sample.

Other protocols are also suitable for obtaining DD and/or PLM images and/or processing them to acquire the ground truth biomarker values of the respective biomarkers.

Water content analysis is performed by weighing the cut pieces before and after drying, and determining the water content as the difference in the weight before and after the drying. The water contents of the pieces are combined to a water content image by assigning the water content of each piece to its respective position within the grid. Thus, water contents in different parts of the sample are obtained in the form of a two-dimensional water content map.

Cartilage integrity maps may be obtained using scoring systems such as the Mankin system, the Osteoarthritis Research Society International (OARSI) system.

An example process of scoring cartilage integrity utilizing any suitable scoring system involves the assessment of stained histological sections from cartilage-on-bone samples. An experienced operator (or experienced operators), well-versed in the influencing factors of scoring, assesses these sections under a microscope and assigns an overall score to each.

For example, in Mankin scoring system, the operator scores the tissue section according to each of the four histological parameters: structural integrity (0-6 points), cellularity (0-3 points), matrix staining (0-4 points), and tidemark integrity (0-2 points). Each parameter has several subcategories. The final score is the sum of all components, where a higher score indicates more severe damage. The final score can be interpreted as follows: 0-2 points indicates normal cartilage, 3-5 early degeneration, 6-10 moderate degeneration, 11-14 is considered advanced structural damage.

Typically, this process is repeated thrice by at least 3 different operators, and the average of all scores per sample is recorded as the final score for that sample. However, the outcome may include a degree of subjectivity due to human operator involvement, nevertheless, this is minimized by averaging the score of several operators. While this repetition minimizes subjectivity, it is not necessary for obtaining a set of training data suitable for the methods described herein.

The methods described herein can be used to generate biomarker images in the form of estimated articular cartilage integrity score maps. In this approach, the training data is prepared as described earlier, preferably extracting sections from diverse areas of the sample. Consequently, scores may be determined at a finer resolution compared to a standard tissue scoring. A final score map may then be interpolated and aligned with the hyperspectral image, allowing the assignment of a score to each pixel. The machine learning model is trained using this data, enabling the estimation of cartilage integrity scores for unseen hyperspectral image cubes.

This parallels the preparation of training data for water content estimation, where initial data are collected at a coarse resolution due to measurement constraints. Subsequently, a more detailed map can be interpolated and aligned with the hyperspectral image. For water content analysis, the minimum size of reliably weighable cartilage pieces is often a limitation. For integrity scoring, limitations include the minimum size of histology sections for reliable scoring and the number of sections that operators can score within a feasible timeframe.

Referring again to FIG. 4, the acquired data may be subjected to post-processing steps 416, 418, 420, 422 before it is used for training the machine learning model 404. The post-processing steps may be performed by the apparatus 100 (see FIG. 1) as part of the method of FIG. 3. The post-processing derives the ground truth images to be used for the training from the non-hyperspectral images. The extent of post-processing depends on the imaging modality and the biomarker, and minimal post-processing may be enough for some combinations of imaging modality and biomarker.

As an example of data processing, the micro-CT data acquired according to the above protocol is subjected to post-processing as follows. The micro-CT images are aligned, cropped, and pixel values of the images are converted into the Hounsfield scale. Next, referring again to FIG. 4, the AC tissue and the subchondral bone are segmented 420. Segmentation enables assignment of labels (annotation) on a pixelwise level to delineate the constituent tissues. FIG. 6 shows an example micro-CT image slice with AC tissue 600 attached to bone 602. The different tissues, in this case the AC 600 and the bone 602, are identified and labelled by the image segmentation.

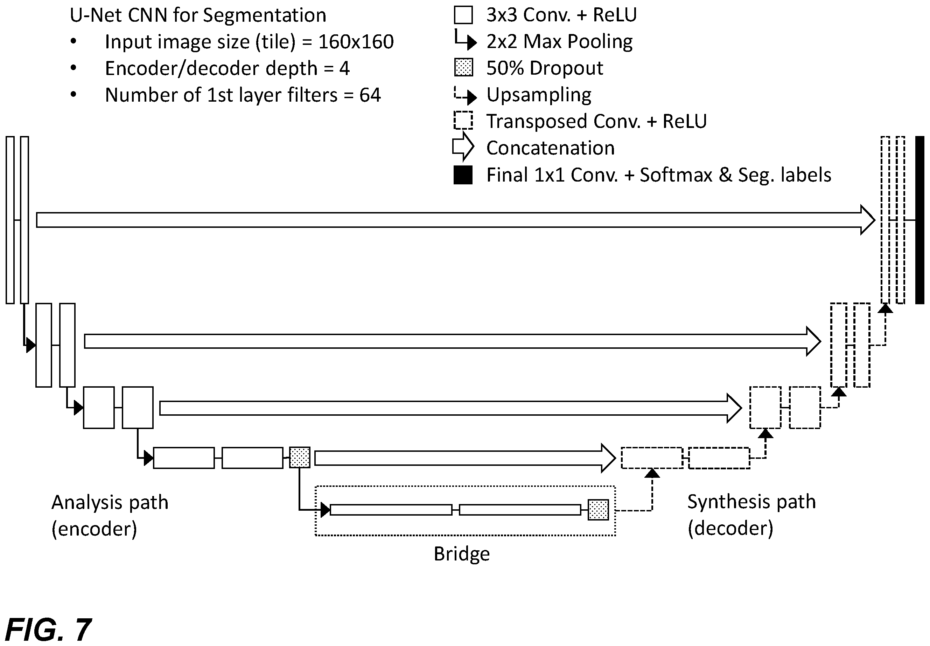

One way for the apparatus 100 (see FIG. 1) to perform the segmentation 422 (se FIG. 4) of the hyperspectral and non-hyperspectral images is with a trained U-Net convolutional neural network (CNN). An example architecture of the U-Net CNN is shown in FIG. 7. The U-Net is a fully convolutional neural network for pixel-wise image segmentation. The architecture includes of a contracting path (the analysis path or encoder) with several stages of convolution filters and pooling followed by an expansive path (the synthesis path or decoder) with the same number of upsampling convolution filters. The down- and upsampling paths together resemble the U shape in the network's name and provide an output label image with the same spatial size as the input. More details on the U-Net can be found in Ronneberger, O., Fischer, P., Brox, T. (2015). U-Net: Convolutional Networks for Biomedical Image Segmentation. In: Navab, N., Hornegger, J., Wells, W., Frangi, A. (eds) Medical Image Computing and Computer-Assisted Intervention - MICCAI 2015. MICCAI 2015. Lecture Notes in Computer Science, vol 9351. Springer, Cham. https://doi.org/10.1007/978-3-319-24574-4_28.

Consequently, using the segmented images or image slices (such as the micro-CT image slice of FIG. 6), the subchondral bone parameters outlined in the above table and the thickness of AC tissue are derived. Referring again to FIG. 6, as an example, the thickness of AC tissue is obtained computationally by the apparatus e.g. by counting pixels of the AC tissue 600 in the vertical direction of the image slice, i.e. by determining the height of the AC tissue 600. Several thickness or height values are obtained along different horizontal positions of the image slice, such as the horizontal positions or cross-sections indicated by the lines 604 and 606. The process of pixel counting is repeated for several horizontal positions of a plurality of image slices of the joint. This results in a pixelwise thickness image, where pixels of the image comprise the thickness values as ground truth biomarker values. The directions (vertical and horizontal) referred to herein are dependent on the orientation of the sample in the image, and the orientation of the sample is preferably automatically detected by the apparatus to select or adjust the pixel counting direction. This means that the thickness of the tissue can be determined also in situations where the measurement orientation is different from the orientation in this example. The thickness values obtained in this manner or in some other way may be used when determining strain (deformation per unit length) in the biomechanical test setup.

Finally, the ground truth image(s), e.g. the post-processed pixelwise thickness image obtained e.g. as described above, is registered 422 (see FIG. 4) to the corresponding hyperspectral image of the joint. Registration establishes a spatial correspondence between the two image modalities so that any pairs of pixels with equal coordinates point to a particular anatomical location, i.e. they are transformed to the same (shared) coordinate system. Image registration may be performed by the apparatus 100 (see FIG. 1) as part of the method of FIG. 3 using any suitable image registration technique or algorithm known to the skilled person. The resolutions of the hyperspectral and ground truth images to be registered may be the same or different, and the skilled person is able to select a suitable registration technique accordingly. For example, the resolution of the water content maps is expected to be lower than that of the corresponding hyperspectral images. The registration facilitates processing of the images by the machine learning model so that the machine learning model is able to effectively learn to predict biomarker values for the hyperspectral images that are fed into it.

The two images whose special correspondence is established during registration may be considered the reference or "fixed" image, and the "moving" image. The process involves finding and applying (i.e. a multiplying the matrices of) a geometrical transformation on the moving image matrix so that it aligns with the fixed image coordinate system. The transformation model is chosen based on the type of geometrical deformation between the two image modalities (the hyperspectral and the ground truth (biomarker map) image, e.g., AC thickness). The hyperspectral image may be the fixed image and the ground truth image the moving image, or vice versa. Due to the use of different between the hyperspectral and ground truth images, the most common deformations include translation, rotation, scaling, and perspective. A projective transformation (defined by a 3x3 homography matrix) accounts for all these distortions. Other distortions, such as radial, barrel, and pincushion distortion, are accounted for in higher order polynomial transformations.

The parameters of the transformation matrix can be determined using e.g. feature-based or intensity-based methods. Feature-based method finds the solution that minimizes the distance between a set of matching points (known as control points) in the two images. These points are found automatically by finding pairs of points with closest features (for example scale invariant feature transform (SIFT) features), or manually. Finding a projective transformation requires a minimum of four non-collinear points. On the other hand, intensity-based methods minimize a global image similarity metric, for example metrics based on correlation or mutual information, between the two images.

For example, for preparing the training data for estimating articular cartilage thickness, the samples were rectangular cartilage-on-bone tissue, extracted from bovine joint, and the sample's four corners were detected as the control points. If the training is performed with in-vivo images, finding matching points on the smooth, homogeneous, and featureless surface of the target tissue (articular cartilage) automatically may be difficult. In this case, distinguishable landmarks can be placed in the scene to be automatically detected as control points during the registration process.

If more than one biomarker map is being estimated, each map (moving image) is registered to the reference hyperspectral image cube separately. The fixed image can be any of the hyperspectral image cube layers, the average of all the layers, or any linear combination of them.

Irrespective of the resolutions of the hyperspectral and ground truth images prior to the registration and of the selection of the registration process, the images should preferably have the same resolution (and preferably also image size and thus pixel count) after registration, so that the machine learning model receives the ground truth image and the corresponding hyperspectral image with exactly same resolution (and size). This can be achieved by the apparatus performing a resolution matching step (e.g. interpolation of the lower-resolution image) in the registration process or prior to the registration process. The latter may be preferred when the resolutions of the two corresponding images have a significant difference. Alternatively, the resolution matching step may be performed twice: first to a near but not exact match of the higher resolution prior to the registration, and then to an exact match during the registration process.

The (registered) hyperspectral images and their corresponding ground truth images form the training set 410. This training data set is used to train the machine learning model 404 to predict biomarker values for pixels of a hyperspectral image in the form of a biomarker image. As a result of the training, the machine learning model becomes able to predict, using hyperspectral images, biomarkers that would not otherwise be as accessible in multimodal and/or longitudinal studies e.g. due to the destructive nature of the techniques needed to obtain these biomarkers.

For the machine learning model, neural networks, especially CNNs, are particularly suitable and efficient in processing images and making use of the spatial information included in them. CNNs jointly exploit the spatial and spectral dimensions of input images; their convolutional layers convolve the input image, abstracting it to feature maps that generate intuitive filters fusing textural and spectral qualities of the target variables. This provides for efficient and accurate prediction of biomarkers from the hyperspectral images.

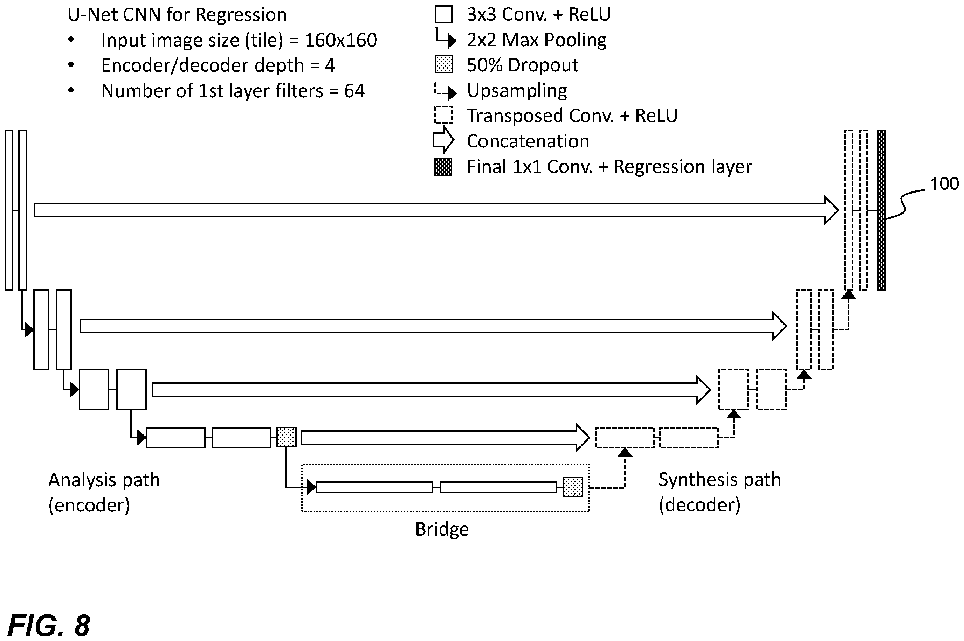

FIG. 8 shows an architecture of a U-net CNN for image regression for use as the machine learning model. The U-net of FIG. 8 differs from the U-net of FIG. 7 used for image segmentation in that instead of a softmax layer and a layer assigning segmentation labels, the final 1x1 convolutional layer of the modified U-net is followed by a (linear) regression layer. This modifies the network to output (numerical) biomarker values instead of classification labels.

A machine learning model implemented as the U-net of FIG. 8 outputs an image with pixels corresponding to predicted biomarker values. Each of the pixels of the output image provides an estimated biomarker value for the corresponding pixel, i.e. the pixel in the same position, of the input hyperspectral image. The convolution kernels of the U-Net (as well as any other CNN, neural network, or even other types of machine learning models) also result in that each biomarker value (pixel) of the output image is based on a plurality of pixels of the hyperspectral image - namely the hyperspectral image pixel that is in the same location or coordinates as the predicted biomarker value is in the output image, and the pixels around or nearby said pixel. In the case of a CNN, the selection of pixels (and their number) to be included in the plurality of pixels depends on the convolution kernel(s) used.

Training of the machine learning model includes determining the model parameters and tuning of the model hyperparameters. For example, if the model is a regressor with Gaussian Radial Basis Function (RBF) kernel, the model parameters are the coefficients and the intercepts (biases) of the regression model, and the hyperparameters are the regularization parameter and the Gaussian kernel width. If the model is a U-net CNN such as the U-Net of FIG. 8, the model parameters are the weights and biases of the network, and the hyperparameters include number of layers, number of filters, filter size, activation function, loss function, learning rate, and batch size among others. Some hyperparameters can be decided by the skilled user and the rest can be decided by running a cross-validation process on the training data set.

For example, when the model is a linear regression with Gaussian RBF kernel, a 2D search grid for possible values of the regularization parameter and the Gaussian kernel width is decided and a cross-validation is performed for each combination of the hyperparameters in the grid search. The final hyperparameters are decided by the apparatus to be the average of the combinations that gave the best performance in each cross-validation fold. Hyperparameters can be tuned simultaneously (such as with a search grid above) or tuned in groups or individually in a sequence. For example, when the model is a U-Net CNN such as the U-Net of FIG. 8, the learning rate and the batch size can be decided first, and then the network architecture parameters, such as number of layers, number of filters, and filter sizes.

When the hyperparameters are decided, the model parameters are decided again, using all the available data with ground truth (training data) and the decided hyperparameter values. This final model can then be used for estimation of future unseen hyperspectral image inputs.

For visualization purposes, the generated biomarker image or images (i.e. derived biomarker maps) can function as standalone images or overlays on various image modalities, offering users additional insights, including the anatomical context of estimated values. To overlay biomarker images, the skilled person or the apparatus selects a suitable colormap and assigns a transparency coefficient for each biomarker. The final image results from blending values for each pixel during visualization.

For example, the estimated thickness and water content of articular cartilage can overlay a grayscale or RGB image of the cartilage surface, captured with a camera probe during arthroscopic surgery. This estimation and overlay process can occur in real-time, facilitating live mapping of tissue biomarkers.

If desired, the different functions discussed herein may be performed in a different order and/or concurrently with other. Furthermore, if desired, one or more of the above-described functions and embodiments may be optional or may be combined.

## Claims

1. A computer-implemented method for processing hyperspectral images of skeletal joints, wherein the method comprises:
obtaining (200) a hyperspectral image depicting at least a part of a skeletal joint of a subject, and
processing (202) the hyperspectral images by a trained machine learning model, wherein the processing comprises:
predicting (204) biomarker values for pixels of the hyperspectral image, and
generating (206) a biomarker image comprising the predicted biomarker values;
wherein the machine learning model has been trained using a training data set to process hyperspectral images of skeletal joints, wherein the training data set comprises a plurality of training images depicting skeletal joints of training subjects, wherein, for each skeletal joint of the training data set, the training data set comprises:
at least one hyperspectral image of at least a part of the joint, and
at least one corresponding ground truth image based on a non-hyperspectral image of at least the part of the joint, wherein the ground truth image comprises one or more ground truth biomarker values for at least the part of the joint also included in at least one hyperspectral image.

2. A computer-implemented method for processing hyperspectral images of skeletal joints, wherein the method comprises:
obtaining (300) a training data set comprising a plurality of training images depicting skeletal joints of training subjects, wherein, for each skeletal joint of the training data set, the training data set comprises:
at least one hyperspectral image of at least a part of the joint, and
at least one corresponding ground truth image based on a non-hyperspectral image of at least the part of the joint, wherein the ground truth image comprises one or more ground truth biomarker values for at least the part of the joint also included in at least one hyperspectral image;
training (302) a machine learning model using the training data set to process hyperspectral images of skeletal joints, wherein the machine learning model is configured to process the hyperspectral images of skeletal joints by:
predicting biomarker values for pixels of a hyperspectral image, and
generating a biomarker image comprising the predicted biomarker values.

3. The method of claim 1 or 2, wherein the predicting comprises:
predicting each biomarker value in the biomarker image based on a plurality of pixels of the hyperspectral image.

4. The method of any one of claims 1 to 3, wherein the biomarker comprises at least one of the following: thickness of articular cartilage, collagen orientation of articular cartilage, tissue composition such as proteoglycan content of articular cartilage and/or collagen content of articular cartilage, subchondral plate thickness, porosity of subchondral bone, mineral density of subchondral bone, subchondral bone volume fraction, water content, cartilage integrity scored with the Mankin system and/or the Osteoarthritis Research Society International system, one or more biomechanical properties such as instantaneous modulus, equilibrium modulus, and/or dynamic modulus.

5. The method of any one of claims 1 to 4, wherein the ground truth images of the training data are based on non-hyperspectral images comprising one or more of the following: computed tomography images such as micro-computed tomography images, densitometry images, polarized light microscopy images, water content maps, biomechanical maps, cartilage integrity maps scored with the Mankin and/or Osteoarthritis Research Society International systems.

6. The method of any one of claims 1 to 5, wherein the ground truth images of the training data are based on computed tomography images such as micro-computed tomography images, and the biomarker comprises one or more of the following: thickness of articular cartilage, subchondral plate thickness, porosity of subchondral bone, mineral density of subchondral bone, subchondral bone volume fraction.

7. The method of any one of claims 1 to 6, wherein the ground truth images of the training data are based on densitometry images, and the biomarker comprises proteoglycan content of articular cartilage.

8. The method of any one of claims 1 to 7, wherein the ground truth images of the training data are based on polarized light microscopy images, and the biomarker comprises collagen orientation of articular cartilage.

9. The method of any one of claims 1 to 8, wherein pixels of the ground truth images of the training data comprise ground truth biomarker values.

10. The method of any one of claims 1 to 9, wherein the machine learning model comprises a neural network, preferably a convolutional neural network.

11. The method of claim 10, wherein the machine learning model comprises a modified U-Net wherein the final layers of the modified U-Net include a final convolutional layer followed by a regression layer.

12. The method of any one of claims 1 to 11, wherein, for each corresponding ground truth image and hyperspectral image of the training data, the hyperspectral image and the ground truth image are registered to the same coordinate system.

13. An apparatus configured to perform the method of any one of claims 1 to 12.

14. A computer program product comprising computer program code configured to, when executed by at least one processor, cause an apparatus or a system to perform the method of any one of claims 1 to 12.
